(19) Europäisches Patentamt
European Patent Office
Office européen des brevets

(11) EP 0 422 751 B1

(12) EUROPÄISCHE PATENTSCHRIFT

(45) Veröffentlichungstag und Bekanntmachung des Hinweises auf die Patenterteilung: **14.08.1996 Patentblatt 1996/33**

(51) Int. Cl.[6]: **C07D 239/52**, C07D 251/20, C07D 239/34, C07D 251/16, A01N 43/54, A01N 43/66

(21) Anmeldenummer: **90250250.9**

(22) Anmeldetag: **04.10.1990**

(54) **Substituierte 2-Pyrimidinyl- und 2-Triazinylessigsäurederivate, Verfahren zu ihrer Herstellung und ihre Verwendung als Mittel mit herbizider, Fungizider und Pflanzenwachstumsregulierender Wirkung**

Substituted 2-pyrimidinyl and 2-triazinylacetic acid derivatives, process for their preparation and their use as agent with herbicidal, fungicidal and plant growth regulating activity

Dérivés d'acide 2-pyrimidinyl et 2-triazinylacétique substitués, procédé pour leur préparation et leur utilisation comme agent herbicide, fongicide et régulateur de croissance des plantes

(84) Benannte Vertragsstaaten:
**AT BE CH DE DK ES FR GB GR IT LI LU NL SE**

(30) Priorität: **09.10.1989 DE 3934020**

(43) Veröffentlichungstag der Anmeldung: **17.04.1991 Patentblatt 1991/16**

(73) Patentinhaber: **Hoechst Schering AgrEvo GmbH 13509 Berlin (DE)**

(72) Erfinder:
- **Harde, Christoph, Dr. W-1000 Berlin 41 (DE)**
- **Nordhoff, Erhard, Dr. W-1000 Berlin 41 (DE)**
- **Krüger, Anita, Dr. W-1000 Berlin 28 (DE)**
- **Krüger, Gabriele, Dr. W-1000 Berlin 21 (DE)**
- **Tarara, Gerhard, Dr. W-1000 Berlin 65 (DE)**
- **Wegner, Peter, Dr. W-1000 Berlin 28 (DE)**
- **Heinrich, Nikolaus, Dr. W-1000 Berlin 33 (DE)**
- **Kötter, Clemens, Dr. W-1000 Berlin 28 (DE)**
- **Johann, Gerhard, Dr. W-1000 Berlin 28 (DE)**
- **Rees, Richard, Dr. W-1000 Berlin 28 (DE)**
- **Jones, Graham Peter GB-Sawston, Cambridge (GB)**

(56) Entgegenhaltungen:
**FR-M- 7 509**

- **JOURNAL OF THE CHEMICAL SOCIETY PERKIN TRANS. I, 1987, Seiten 2523-2529, Londen, GB; T. NISHIO et al.: "Photochemical reactions of pyrimidinethioneswith alkenes"**
- **CHEMICAL ABSTRACTS, Band 83, 1975, Seite 661, Zusammenfassung Nr. 79200k,Columbus, Ohio, US; R.T. SANE et al.: "Synthesis of new copper(II) complexes ofthe derivatives of 2,4,6-trichloro-s-triazine",& CURR. SCI. 1975, 44(7), 220-1**
- **CHEMICAL ABSTRACTS, Band 91, 1979, Seite 659, Zusammenfassung Nr. 175289g,Columbus, Ohio, US; H. YAMANAKA et al.: "Studies on pyrimidine derivatives. XI.Reaction of ethoxycarbonylacetamidine with beta-dicarbonyl compounds",& YAKUGAKU ZASSHI 1979, 99(4), 342-8**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 422 751 B1

Printed by Rank Xerox (UK) Business Services
2.13.0/3.4

## Beschreibung

Die Erfindung betrifft neue substituierte 2-Pyrimidinyl- und 2-Triazinylessigsäurederivate, Verfahren zu ihrer Herstellung und ihre Verwendung als Mittel mit herbizider, fungizider und pflanzenwachstumsregulierender Wirkung.

Es ist bereits bekannt, daß Pyrimidinderivate eine herbizide Wirkung besitzen (EP-Anmeldungen 0 223 406, 0 249 707, 0 249 708, 0 287 072 und 0 287 079). Häufig ist jedoch die Herbizidwirkung der bekannten Verbindungen nicht ausreichend, beziehungsweise es treten bei entsprechender Herbizidwirkung Selektivitätsprobleme in landwirtschaftlichen Hauptkulturen auf.

Aufgabe der vorliegenden Erfindung ist die Bereitstellung von neuen Verbindungen, die diese Nachteile nicht aufweisen und die in ihren biologischen Eigenschaften den bisher bekannten Verbindungen überlegen sind.

Es wurde nun gefunden, daß substituierte 2-Pyrimidinyl- und 2-Triazinylessigsäurederivate der allgemeinen Formel I

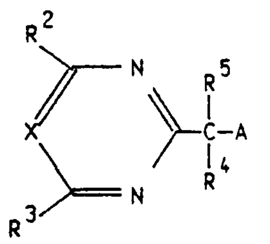

(I) ,

in der

A die Gruppe -CO-$OR^1$,

$R^1$ ein Wasserstoffatom, einen $C_1$-$C_{12}$-Alkylrest, einen durch Hydroxy, Halogen, $C_1$-$C_4$-Alkoxy, $C_1$-$C_4$-Alkylthio, $C_3$-$C_6$-Cycloalkyl, Benzyl, Furyl, Tetrahydrofuryl, Phenyl, Halogenphenyl, $C_1$-$C_4$-Alkylphenyl, $C_1$-$C_4$-Alkoxyphenyl, Nitrophenyl, Cyano, Carboxyl, $C_1$-$C_4$-Alkoxycarbonyl, $C_1$-$C_4$-Alkylamino, Di-$C_1$-$C_4$-alkylamino, Tri-$C_1$-$C_4$-alkylammoniumhalogenid, $C_2$-$C_5$-Alkylenimino oder Di-$C_1$-$C_4$-alkylmethyleniminooxy substituierten $C_1$-$C_{12}$-Alkylrest, einen durch ein oder mehrere Sauerstoffatome oder Schwefelatome unterbrochenen $C_2$-$C_{12}$-Alkylrest, einen durch ein oder mehrere Sauerstoffatome oder Schwefelatome unterbrochenen und durch Hydroxy, Halogen, $C_1$-$C_4$-Alkoxy, $C_1$-$C_4$-Alkylthio, $C_3$-$C_6$-Cycloalkyl, Benzyl, Furyl, Tetrahydrofuryl, Phenyl, Halogenphenyl, $C_1$-$C_4$-Alkylphenyl, $C_1$-$C_4$-Alkoxyphenyl, Nitrophenyl, Cyano, Carboxyl, $C_1$-$C_4$-Alkoxycarbonyl, $C_1$-$C_4$-Alkylamino oder Di-$C_1$-$C_4$-alkylamino substituierten $C_2$-$C_{12}$-Alkylrest, einen $C_3$-$C_8$-Alkenylrest, einen durch $C_1$-$C_3$-Alkoxy, Phenyl oder Halogen substituierten $C_3$-$C_8$-Alkenylrest, einen $C_3$-$C_8$-Alkinylrest, einen durch $C_1$-$C_3$-Alkoxy, Phenyl oder Halogen substituierten $C_3$-$C_8$-Alkinylrest, einen $C_3$-$C_6$-Cycloalkylrest, einen durch Methyl substituierten $C_3$-$C_6$-Cycloalkylrest oder einen Di-$C_1$-$C_4$-Alkylmethyleniminorest oder einen $C_3$-$C_6$-Cycloalkyleniminorest, mit der Maßgabe, daß $R^1$ nicht der Ethylrest ist, wenn $R^5$ der Benzylrest ist,

$R^2$ einen $C_1$-$C_4$-Alkoxyrest,

$R^3$ einen $C_1$-$C_4$-Alkoxyrest,

$R^4$ ein Wasserstoffatom,

$R^5$ einen $C_1$-$C_{10}$-Alkylrest, einen ein- oder mehrfach, gleich oder verschieden durch $C_1$-$C_4$-Alkoxy, $C_1$-$C_4$-Alkylthio, Amino, $C_1$-$C_4$-Alkylamino, Di-$C_1$-$C_4$-alkylamino, Nitro, Halogen, Phenyl oder Halogenphenyl substituierten $C_1$-$C_{10}$-Alkylrest, einen $C_2$-$C_{10}$-Alkenylrest, einen ein- oder mehrfach, gleich oder verschieden durch $C_1$-$C_4$-Alkoxy, $C_1$-$C_4$-Alkylthio, Amino, $C_1$-$C_4$-Alkylamino, Di-$C_1$-$C_4$-alkylamino, Nitro, Halogen oder Phenyl substituierten $C_2$-$C_{10}$-Alkenylrest, einen $C_2$-$C_{10}$-Alkinylrest, einen ein- oder mehrfach, gleich oder verschieden durch $C_1$-$C_4$-Alkoxy, $C_1$-$C_4$-Alkylthio, Amino, $C_1$-$C_4$-Alkylamino, Di-$C_1$-$C_4$-alkylamino, Nitro, Halogen oder Phenyl substituierten $C_2$-$C_{10}$-Alkinylrest, einen $C_3$-$C_8$-Cycloalkylrest, einen ein- oder mehrfach, gleich oder verschieden durch $C_1$-$C_4$-Alkyl, $C_1$-$C_4$-Alkoxy, $C_1$-$C_4$-Alkylthio, Amino, $C_1$-$C_4$-Alkylamino, Di-$C_1$-$C_4$-alkylamino, Nitro, Trifluormethyl, Halogen oder Phenyl substituierten $C_3$-$C_8$-Cycloalkylrest, einen $C_4$-$C_8$-Cycloalkenylrest oder einen ein- oder mehrfach, gleich oder verschieden durch $C_1$-$C_4$-Alkyl, $C_1$-$C_4$-Alkoxy, $C_1$-$C_4$-Alkylthio, Amino, $C_1$-$C_4$-Alkylamino, Di-$C_1$-$C_4$-alkylamino, Nitro, Trifluormethyl, Halogen oder Phenyl substituierten $C_4$-$C_8$-Cycloalkenylrest und

X eine Methingruppe oder ein Stickstoffatom,

bedeuten, sowie deren Alkalimetallsalze, Erdalkalimetallsalze, Ammoniumsalze und organisch substituierten Ammoniumsalze und deren optisch aktive Isomeren eine interessante herbizide, fungizide und pflanzenwachstumsregulierende Wirkung zeigen.

Die Bezeichnung Halogen umfaßt Fluor, Chlor, Brom und Jod. Unter der Bezeichnung Alkalimetall ist Lithium, Natrium und Kalium, unter der Bezeichnung Erdalkalimetall Calcium, Strontium und Barium zu verstehen.

Die erfindungsgemäßen Verbindungen der allgemeinen Formel I können zum Beispiel hergestellt werden, indem man

A) Verbindungen der allgemeinen Formel II

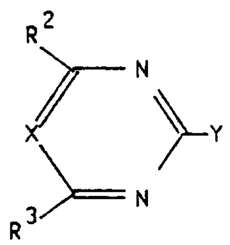

(II) ,

in der $R^2$, $R^3$ und X die unter der allgemeinen Formel I genannten Bedeutungen haben und Y für ein Halogenatom, eine Alkylsulfonylgruppe oder eine Phenylsulfonylgruppe steht, mit Verbindungen der allgemeinen Formel III

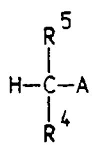

(III) ,

in der A, $R^4$ und $R^5$ die unter der allgemeinen Formel I genannten Bedeutungen haben, in einem geeigneten Lösungsmittel in Gegenwart einer geeigneten Base umsetzt, oder

B) Verbindungen der allgemeinen Formel IV

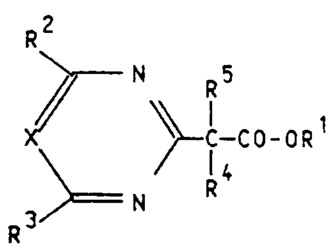

(IV) ,

in der $R^1$, $R^2$, $R^3$, $R^4$, $R^5$ und X die unter der allgemeinen Formel I angegebenen Bedeutungen haben, $R^1$ aber nicht für ein Wasserstoffatom steht, in einem geeigneten polaren Lösungsmittel mit einer Alkalimetallbase oder einer Erdalkalimetallbase zu Verbindungen der allgemeinen Formel V

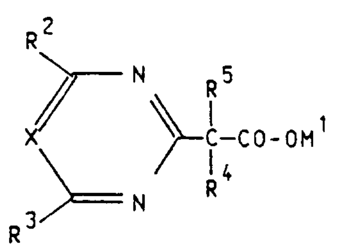

(V) ,

in der $R^2$, $R^3$, $R^4$, $R^5$ und X die unter der allgemeinen Formel I angegebenen Bedeutungen haben und $M^1$ für ein Alkalimetallatom oder ein Äquivalent eines Erdalkalimetallatoms steht, umsetzt, oder

C) Verbindungen der allgemeinen Formel V

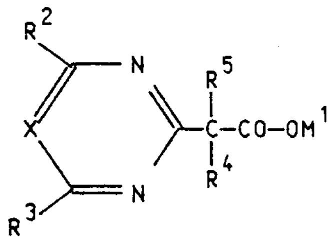

(V) ,

in der $R^2$, $R^3$, $R^4$, $R^5$, $M^1$ und X die unter der allgemeinen Formel V angegebenen Bedeutungen haben, mit einer geeigneten Säure in einem geeigneten Lösungsmittel zu Verbindungen der allgemeinen Formel VI

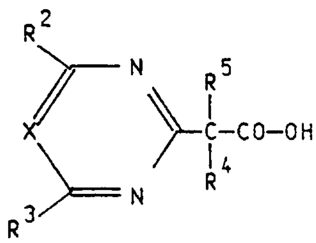

(VI) ,

in der $R^2$, $R^3$, $R^4$, $R^5$ und X die unter der allgemeinen Formel I angegebenen Bedeutungen haben, umsetzt, oder

D) Verbindungen der allgemeinen Formel VI

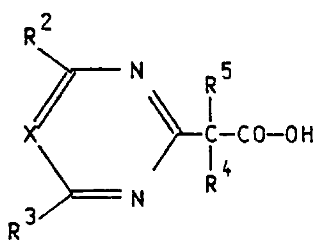

(VI) ,

in der $R^2$, $R^3$, $R^4$, $R^5$ und X die unter der allgemeinen Formel I angegebenen Bedeutungen haben, mit einer geeigneten Base in einem geeigneten Lösungsmittel zu einer Verbindung der allgemeinen Formel VII

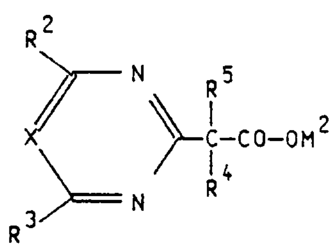

(VII) ,

in der $R^2$, $R^3$, $R^4$, $R^5$ und X die unter der allgemeinen Formel I angegeben Bedeutungen haben und $M^2$ für ein Alkalimetallatom, ein Äquivalent eines Erdalkalimetallatom, ein Ammoniumion oder eine organisch substituierte Ammoniumgruppe steht, umsetzt, oder

E) eine Verbindung der allgemeinen Formel VIII

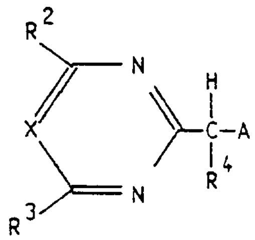

(VIII) ,

in der A, $R^2$, $R^3$, $R^4$ und X die unter der allgemeinen Formel I angegebenen Bedeutungen haben, mit einer Verbindung der allgemeinen Formel IX

$$R^5 - Z \qquad (IX),$$

in der $R^5$ die unter der allgemeinen Formel I angegebenen Bedeutungen hat und Z für ein Halogenatom, eine Alkylsulfonyloxygruppe, eine Phenylsulfonyloxygruppe oder eine Gruppe $-O-SO_2-O-$Alkyl steht, in einem geeigneten Lösungsmittel in Anwesenheit einer geeigneten Base umsetzt.

Die einzelnen Verfahrensvarianten werden vorzugsweise in Gegenwart eines Verdünnungsmittels durchgeführt. Zu diesem Zweck können sämtliche gegenüber den verwendeten Reagenzien inerte Lösungsmittel verwendet werden.

Beispiele für solche Lösungsmittel beziehungsweise Verdünnungsmittel sind Wasser, aliphatische, alicyclische und aromatische Kohlenwasserstoffe, die jeweils gegebenenfalls chloriert sein können, wie zum Beispiel Hexan, Cyclohexan, Petrolether, Ligroin, Benzol, Toluol, Xylol, Methylenchlorid, Chloroform, Kohlenstofftetrachlorid, Ethylenchlorid und Trichlorethylen, Ether, wie zum Beispiel Diisopropylether, Dibutylether, Propylenoxid, Dioxan und Tetrahydrofuran, Ketone, wie zum Beispiel Aceton, Methylethylketon, Methylisopropylketon und Methylisobutylketon, Nitrile, wie zum Beispiel Acetonitril und Propionitril, Alkohole, wie zum Beispiel Methanol, Ethanol, Isopropanol, Butanol und Ethylenglycol, Ester, wie zum Beispiel Ethylacetat und Amylacetat, Säureamide, wie zum Beispiel Dimethylformamid und Dimethylacetamid, Sulfoxide und Sulfone, wie zum Beispiel Dimethylsulfoxid und Sulfolan, und Basen, wie zum Beispiel Pyridin.

Die Gegenwart eines Reaktionskatalysators kann von Vorteil sein. Als Katalysatoren sind Kaliumjodid und Oniumverbindungen geeignet, wie quaternäre Ammonium-, Phosphonium- und Arsoniumverbindungen sowie Sulfoniumverbindungen. Ebenfalls geeignet sind Polyglycolether, insbesondere cyclische, wie zum Beispiel 18-Krone-6, und tertiäre Amine, wie zum Beispiel Tributylamin. Bevorzugte Verbindungen sind quaternäre Ammoniumverbindungen, wie zum Beispiel Benzyltriethylammoniumchlorid und Tetrabutylammoniumbromid.

Die Reaktionen lassen sich bei dem Druck der Umgebung durchführen, wenngleich sie auch bei erhöhtem beziehungsweise vermindertem Druck durchgeführt werden können.

Für die Verfahrensvariante A) können als Lösungsmittel bevorzugt aprotische Solventien, wie zum Beispiel Benzol, Toluol, Xylol, Tetrahydrofuran, Diethylether, Hexan, Dimethylformamid oder Dimethylsulfoxid verwendet werden.

Als Basen können zum Beispiel Natriumhydrid, Kaliumtertiärbutylat oder Lithiumdiisopropylamid verwendet werden.

Die Reaktionstemperatur liegt zwischen -78°C und der Siedetemperatur des jeweiligen Lösungsmittels beziehungsweise Lösungsmittelgemisches.

Die Reaktionszeit betragt 5 Minuten bis 48 Stunden, vorzugsweise 0,5 bis 24 Stunden.

Die Verbindungen der allgemeinen Formel II sind in der Literatur beschrieben, oder können nach literaturanalogen Verfahren hergestellt werden.

Die Verfahrensvarianten B) und C) werden bevorzugt in Alkoholen, wie Methanol, Ethanol oder Isopropanol, in Ketonen, wie Aceton oder Methylketonen, in Wasser oder in einer Mischung aus Wasser und einem polaren Lösungsmittel durchgeführt.

Als Base kann ein Carbonat, wie Natriumcarbonat, Kaliumcarbonat oder Calciumcarbonat und ein Metallhydroxid, wie Natriumhydroxid oder Kaliumhydroxid, verwendet werden.

Der Temperaturbereich der Reaktion liegt zwischen Raumtemperatur und dem Siedepunkt des jeweiligen Lösungsmittels oder Losungsmittelgemisches. Die Reaktionzeit betragt 0,5 bis 36 Stunden.

Falls $R^1$ in der allgemeinen Formel IV eine Benzylgruppe bedeutet, kann eine Verbindung der allgemeinen Formel VI auch durch katalytische Reduktion (Hydrierung) erhalten werden.

Für die Verfahrensvariante D) können als Lösungsmittel Kohlenwasserstoffe, wie Benzol, Toluol oder Xylol, halogenierte Kohlenwasserstoffe, wie Methylenchlorid oder Chloroform, Alkohole, wie Methanol, Ethanol oder Isopropanol,

Ether, wie Ethylether, Isopropylether, Tetrahydrofuran oder 1,4-Dioxan, Ketone, wie Aceton oder Methylethylketon, Ester, wie Methylacetat oder Ethylacetat, oder Nitrile, wie Acetonitril, verwendet werden.

Als Base kann ein Alkalimetall, wie Natrium oder Kalium, ein Alkalimetall- oder Erdalkalimetallhydrid, wie Natriumhydrid, Kaliumhydrid oder Calciumhydrid, ein Carbonat, wie Natriumcarbonat, Kaliumcarbonat oder Calciumcarbonat, ein Metallhydroxid, wie Natriumhydroxid oder Kaliumhydroxid, oder Ammoniak verwendet werden. Als organische Base kann ein Alkylamin (primäres Amin), ein Dialkylamin (sekundäres Amin), ein Trialkylamin (tertiäres Amin) oder Pyridin verwendet werden.

Der Temperaturbereich der Reaktion liegt zwischen Raumtemperatur und dem Siedepunkt des jeweiligen Lösungsmittels oder Lösungsmittelgemisches. Die Reaktionszeit beträgt 5 Minuten bis 10 Stunden.

Für die Verfahrensvariante E) können als Lösungsmittel bevorzugt aprotische Solventien, wie zum Beispiel Benzol, Toluol, Xylol, Tetrahydrofuran, Diethylether, Hexan, Dimethylformamid oder Dimethylsulfoxid verwendet werden.

Als Basen können zum Beispiel Natriumhydrid, Kaliumtertiärbutylat oder Lithiumdiisopropylamid verwendet werden.

Die Reaktionstemperatur liegt zwischen -78°C und der Siedetemperatur des jeweiligen Lösungsmittels beziehungsweise Lösungsmittelgemisches.

Die Reaktionszeit beträgt 1 bis 24 Stunden.

Die nach den oben genannten Verfahren hergestellten erfindungsgemäßen Verbindungen können nach den üblichen Verfahren aus dem Reaktionsgemisch isoliert werden, beispielsweise durch Abdestillieren des eingesetzten Lösungsmittels bei normalem oder vermindertem Druck, durch Ausfällen mit Wasser oder durch Extraktion.

Ein erhöhter Reinheitsgrad kann in der Regel durch säulenchromatographische Aufreinigung sowie durch fraktionierte Destillation oder Kristallisation erhalten werden.

Die erfindungsgemäßen Verbindungen stellen in der Regel farb- und geruchlose Flüssigkeiten sowie Kristalle dar, die löslich in Wasser, wenig löslich in aliphatischen Kohlenwasserstoffen, wie Petrolether, Hexan, Pentan und Cyclohexan, gut löslich in halogenierten Kohlenwasserstoffen, wie Chloroform, Methylenchlorid und Tetrachlorkohlenstoff, aromatischen Kohlenwasserstoffen, wie Benzol, Toluol und Xylol, Ethern, wie Diethylether, Tetrahydrofuran und Dioxan, Carbonsäurenitrilen, wie Acetonitril, Alkoholen, wie Methanol und Ethanol, Carbonsäureamiden, wie Dimethylformamid, und Sulfoxiden, wie Dimethylsulfoxid, sind.

Die erfindungsgemäßen Verbindungen zeigen eine gute herbizide Wirkung bei breitblättrigen Unkräutern und Gräsern. Ein selektiver Einsatz der erfindungsgemäßen Wirkstoffe ist in verschiedenen Kulturen möglich, zum Beispiel in Raps, Rüben, Sojabohnen, Baumwolle, Reis, Gerste, Weizen und anderen Getreidearten. Dabei sind einzelne Wirkstoffe als Selektivherbizide in Rüben, Baumwolle, Soja und Getreide besonders geeignet. Ebenso können die Verbindungen zur Unkrautbekämpfung in Dauerkulturen, wie zum Beispiel Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, und zur selektiven Unkrautbekämpfung in einjährigen Kulturen eingesetzt werden.

Die erfindungsgemäßen Verbindungen können zum Beispiel bei den folgenden Pflanzengattungen verwendet werden:

Dikotyle Unkräuter der Gattungen Sinapis, Lepidium, Galium, Stellaria, Matricaria, Anthemis, Galinsoga, Chenopodium, Brassica, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Lamium, Veronica, Abutilon, Datura, Viola, Galeopsis, Papaver, Centaurea und Chrysanthemum.

Monokotyle Unkräuter der Gattungen Avena, Alopecurus, Echinochloa, Setaria, Panicum, Digitaria, Poa, Eleusine, Brachiaria, Lolium, Bromus, Cyperus, Agropyron, Sagittaria, Monochoria, Fimbristylis, Eleocharis, Ischaemum und Apera.

Die Aufwandmengen schwanken je nach Anwendungsart im Vor- und Nachauflauf in Grenzen zwischen 0,001 bis 5 kg/ha.

Die erfindungsgemäßen Wirkstoffe können auch als Defoliant, Desiccant und als Krautabtötungsmittel verwendet werden. Sie beeinflussen auch das Pflanzenwachstum und können deshalb zur Wachstumsbeeinflussung von Kulturpflanzen eingesetzt werden. Einige dieser Wirkstoffe zeigen auch eine fungizide Wirkung.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden. Sofern eine Verbreiterung des Wirkungsspektrums beabsichtigt ist, können auch andere Herbizide zugesetzt werden.

Beispielsweise eignen sich als herbizid wirksame Mischungspartner diejenigen Wirkstoffe, die in Weed Abstracts, Vol. 38, No. 3, 1989, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe, wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

**A) Spritzpulver**

1.)

| | |
|---|---|
| 25 Gewichtsprozent | Wirkstoff |
| 60 Gewichtsprozent | Kaolin |
| 10 Gewichtsprozent | Kieselsäure |
| 5 Gewichtsprozent | einer Mischung aus dem Calciumsalz der Ligninsulfonsäure und dem Natriumsalz des N-Methyl-N-oleyl-taurins |

2.)

| | |
|---|---|
| 40 Gewichtsprozent | Wirkstoff |
| 25 Gewichtsprozent | Tonmineralien |
| 25 Gewichtsprozent | Kieselsäure |
| 10 Gewichtsprozent | einer Mischung aus dem Calciumsalz der Ligninsulfonsäure und Alkylphenylpolyglycolethern |

**B) Paste**

| | |
|---|---|
| 45 Gewichtsprozent | Wirkstoff |
| 5 Gewichtsprozent | Natriumaluminiumsilikat |
| 15 Gewichtsprozent | Cetylpolyglycolether mit 8 Mol Ethylenoxid |
| 2 Gewichtsprozent | Spindelöl |
| 10 Gewichtsprozent | Polyethylenglycol |
| 23 Gewichtsprozent | Wasser |

**C) Emulsionskonzentrat**

| | |
|---|---|
| 25 Gewichtsprozent | Wirkstoff |
| 15 Gewichtsprozent | Cyclohexanon |
| 55 Gewichtsprozent | Xylol |
| 5 Gewichtsprozent | einer Mischung aus dem Calciumsalz der Dodecylbenzolsulfonsäure und Nonylphenylpolyoxyethylen |

Die nachfolgenden Beispiele erläutern die Herstellung der erfindungsgemäßen Verbindungen:

Beispiel 1

**2-(4,6-Dimethoxypyrimidin-2-yl)-pentansäureethylester**

4,2 ml (30 mmol) Diisopropylamin werden in 100 ml Tetrahydrofuran unter Stickstoffatmosphäre bei -78°C mit 19 ml (30 mmol) 1,6 M Butyllithium, gelöst in Hexan, versetzt. Es wird 30 Minuten nachgerührt. Bei -60°C werden dann 3,5 g (27 mmol) Pentansäureethylester, die in 20 ml Tetrahydrofuran gelöst sind, zugetropft. Man rührt eine Stunde nach und gibt 5,9 g (27 mmol) 4,6-Dimethoxy-2-methylsulfonylpyrimidin portionsweise zu. Die Reaktionstemperatur steigt innerhalb von zwei Stunden auf +10°C. Das Reaktionsgemisch wird dann mit gesättigter Ammoniumchlorid- und Natriumchloridlösung gewaschen. Die wäßrigen Phasen werden mit Essigester extrahiert. Die organischen Phasen werden über Magnesiumsulfat getrocknet und eingeengt. Das erhaltene gelbe Öl wird schließlich an Kieselgel mit Hexan/Essigestergemischen (0 bis 20 % Essigester) gereinigt.

Ausbeute: 3,2 g = 42 % der Theorie
$n_D^{20}$: 1,4824

Beispiel 2

**2-(4,6-Dimethoxypyrimidin-2-yl)-pentansäure**

2,2 g (8 mmol) 2-(4,6-Dimethoxypyrimidin-2-yl)-pentansäureethylester werden in 20 ml Methanol und 3 ml 5 N Natronlauge über Nacht bei Raumtemperatur gerührt. Man engt ein, nimmt mit 10 ml Wasser auf, extrahiert mit 50 ml Essigester und säuert die wäßrige Phase mit 2 N Salzsäure auf pH 5 an. Dann wird die wäßrige Phase dreimal mit Ether extrahiert, die vereinigten Etherphasen werden über Magnesiumsulfat getrocknet und eingeengt.

Ausbeute: 1,7 g = 85 % der Theorie
Fp.: 71-73°C (Zersetzung)

Beispiel 3

**2,4-Dimethyl-2-(4,6-dimethoxy-s-triazin-2-yl)-pentansäuremethylester**

0,5 g (17 mmol) Natriumhydrid (80 % in Öl) werden bei Raumtemperatur in einer Stickstoffatmosphäre zu einer Lösung von 3,96 g (14 mmol) 2-(4,6-Dimethoxytriazin-2-yl)-4-methylpentansäuremethylester in 35 ml Dimethylformamid gegeben. Es wird eine Stunde bei Raumtemperatur gerührt und anschließend 2,0 g (16 mmol) Dimethylsulfat zugetropft. Nach 2,5 Stunden Reaktionszeit bei Raumtemperatur gibt man die Reaktionsmischung auf 200 ml Wasser und extrahiert zweimal mit Essigester. Die vereinigten organischen Phasen werden mit gesättigter Natriumchloridlösung gewaschen, über Magnesiumsulfat getrocknet und eingeengt. Das Rohprodukt wird chromatographisch an Kieselgel mit Hexan/Essigestergemischen (0 bis 20 % Essigester) gereinigt.

Ausbeute: 2,3 g = 62 % der Theorie
$n_D^{20}$: 1,4775

In analoger Weise wurden auch die folgenden erfindungsgemäßen Verbindungen hergestellt:

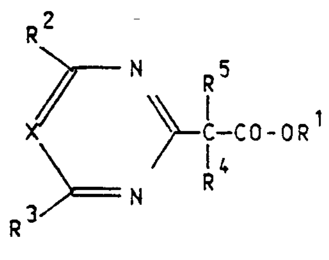

| Beispiel Nr. | $R^1$ | $R^2$ | $R^3$ | $R^4$ | $R^5$ | X | Physikalische Konstante |
|---|---|---|---|---|---|---|---|
| 4 | $CH_2CH_3$ | $OCH_3$ | $OCH_3$ | H | $CH(CH_3)_2$ | CH | $n_D^{20}$: 1,4794 |
| 5 | H | $OCH_3$ | $OCH_3$ | H | $CH(CH_3)_2$ | CH | Fp.: 109-110°C (Zersetzung) |
| 6 | $CH_3$ | $OCH_3$ | $OCH_3$ | H | Cyclo-hexyl | CH | $n_D^{20}$: 1,5028 |
| 7 | $CH_2CH_3$ | $OCH_3$ | $OCH_3$ | H | Cyclo-hexyl | CH | $n_D^{20}$: 1,4982 |
| 8 | H | $OCH_3$ | $OCH_3$ | H | Cyclo-hexyl | CH | Fp.: 88-89°C (Zersetzung) |
| 9 | $CH_3$ | $OCH_3$ | $OCH_3$ | H | $C(CH_3)_3$ | CH | $n_D^{20}$: 1,4868 |
| 10 | $CH_2CH_3$ | $OCH_3$ | $OCH_3$ | H | $(CH_2)_4CH_3$ | CH | $n_D^{20}$: 1,4755 |
| 11 | H | $OCH_3$ | $OCH_3$ | H | $(CH_2)_4CH_3$ | CH | $n_D^{20}$: 1,4920 |
| 12 | $CH_3$ | $OCH_3$ | $OCH_3$ | H | $(CH_2)_2SCH_3$ | CH | $n_D^{20}$: 1,5175 |
| 13 | $CH_2CH_3$ | $OCH_3$ | $OCH_3$ | H | $CH_2CH(CH_3)_2$ | CH | $n_D^{20}$: 1,4773 |

| Beispiel Nr. | $R^1$ | $R^2$ | $R^3$ | $R^4$ | $R^5$ | X | Physikalische Konstante |
|---|---|---|---|---|---|---|---|
| 15 | $CH_2CH_3$ | $OCH_3$ | $OCH_3$ | H | $CH(CH_3)_2$ | N | $n_D^{20}$: 1,4796 |
| 19 | $CH_3$ | $OCH_3$ | $OCH_3$ | $CH_3$ | $(CH_2)_2CH_3$ | CH | $n_D^{20}$: 1,4829 |
| 20 | $CH_2CH_3$ | $OCH_3$ | $OCH_3$ | H | $CH_2CH(CH_3)_2$ | N | $n_D^{20}$: 1,4740 |
| 21 | $CH_2CH_3$ | $OCH_3$ | $OCH_3$ | H | $CH_3$ | CH | $n_D^{20}$: 1,4808 |
| 22 | $CH_3$ | $OCH_3$ | $OCH_3$ | H | $(CH_2)CH_3$ | CH | $n_D^{20}$: 1,4834 |
| 23 | $CH_3$ | $OCH_3$ | $OCH_3$ | H | $CH_2(CH_3)_2$ | N | $n_D^{20}$: 1,4834 |
| 24 | H | $OCH_3$ | $OCH_3$ | H | $C(CH_3)_3$ | N | Fp.: 82-83°C (Zersetzung) |
| 25 | $CH_3$ | $OCH_3$ | $OCH_3$ | H | $CH_2CH{=}CH_2$ | N | $n_D^{20}$: 1,4966 |
| 26 | $CH_2CH_3$ | $OCH_3$ | $OCH_3$ | H | $CH_2CH{=}CH_2$ | N | $n_D^{20}$: 1,4882 |
| 27 | H | $OCH_3$ | $OCH_3$ | H | $CH_2CH{=}CH_2$ | N | Fp.: 71-73°C |
| 28 | H | $OCH_3$ | $OCH_3$ | H | $CH_2CH(CH_3)_2$ | N | Fp.: 86-88°C |
| 29 | $CH_2CH_3$ | $OCH_3$ | $OCH_3$ | H | $(CH_2)_2C{\equiv}CH$ | N | $n_D^{20}$: 1,4903 |
| 30 | H | $OCH_3$ | $OCH_3$ | H | $(CH_2)_2C{\equiv}CH$ | N | Fp.: 73-75°C (Zersetzung) |

| Beispiel Nr. | $R^1$ | $R^2$ | $R^3$ | $R^4$ | $R^5$ | X | Physikalische Konstante |
|---|---|---|---|---|---|---|---|
| 31 | $CH_3$ | $OCH_3$ | $OCH_3$ | H | $CH(CH_3)_2$ | N | $n_D^{20}$: 1,4815 |
| 32 | $CH_2(CH_3)_2$ | $OCH_3$ | $OCH_3$ | H | $CH(CH_3)_2$ | N | $n_D^{20}$: 1,4744 |
| 33 | Tetra-hydro-furfuryl | $OCH_3$ | $OCH_3$ | H | $CH(CH_3)_2$ | N | $n_D^{20}$: 1,4946 |
| 34 | $CH_2C{\equiv}CH$ | $OCH_3$ | $OCH_3$ | H | $CH(CH_3)_2$ | N | $n_D^{20}$: 1,4922 |
| 35 | $CH_2CH{=}C{-}(CH_3)C{\equiv}CH$ | $OCH_3$ | $OCH_3$ | H | $CH(CH_3)_2$ | N | $n_D^{20}$: 1,5021 |
| 36 | $H_3NCH(CH_3)_2$ | $OCH_3$ | $OCH_3$ | H | $CH(CH_3)_2$ | N | Fp.: 127°C (Zersetzung) |
| 37 | $N{=}C(CH_3)_2$ | $OCH_3$ | $OCH_3$ | H | $CH(CH_3)_2$ | N | $n_D^{20}$: 1,4895 |
| 38 | $CH_2OCH_3$ | $OCH_3$ | $OCH_3$ | H | $CH(CH_3)_2$ | N | $n_D^{20}$: 1,4782 |
| 39 | $CH_2C{\equiv}CH$ | $OCH_3$ | $OCH_3$ | H | $C(CH_3)_3$ | N | Fp.: 72-74°C (Zersetzung) |
| 40 | $CH_2CH{=}C{-}(CH_3)C{\equiv}CH$ | $OCH_3$ | $OCH_3$ | H | $C(CH_3)_3$ | N | $n_D^{20}$: 1,5049 |

| Beispiel Nr. | $R^1$ | $R^2$ | $R^3$ | $R^4$ | $R^5$ | X | Physikalische Konstante |
|---|---|---|---|---|---|---|---|
| 48 | $CH_3$ | $OCH_3$ | $OCH_3$ | H | $(CH_2)_3CH_3$ | N | $n_D^{20}$: 1,4716 |
| 49 | $CH_3$ | $OCH_3$ | $OCH_3$ | H | $CH_2CH(CH_3)_2$ | N | $n_D^{20}$: 1,4794 |
| 53 | $CH_3$ | $OCH_3$ | $OCH_3$ | H | $(CH_2)_3CH_3$ | CH | $n_D^{20}$: 1,3769 |
| 54 | $CH_2CH_3$ | $OCH_3$ | $OCH_3$ | H | $(CH_2)_3CH_3$ | CH | $n_D^{20}$: 1,4781 |
| 55 | H | $OCH_3$ | $OCH_3$ | H | $(CH_2)_3CH_3$ | CH | Fp.: 71-72°C |
| 56 | $N=C(CH_3)_2$ | $OCH_3$ | $OCH_3$ | H | $(CH_2)_3CH_3$ | CH | $n_D^{20}$: 1,4972 |
| 58 | $CH_3$ | $OCH_3$ | $OCH_3$ | H | $(CH_2)_2CH_3$ | N | $n_D^{20}$: 1,4844 |
| 59 | $CH_3$ | $OCH_3$ | $OCH_3$ | H | Cyclo-propyl-methyl | CH | $n_D^{20}$: 1,4973 |

| Beispiel Nr. | $R^1$ | $R^2$ | $R^3$ | $R^4$ | $R^5$ | X | Physikalische Konstante |
|---|---|---|---|---|---|---|---|
| 60 | $CH_3$ | $OCH_3$ | $OCH_3$ | H | $CH(CH_3)-CH_2CH_3$ | CH | $n_D^{20}$: 1,4846 |
| 61 | $CH_2CH_3$ | $OCH_3$ | $OCH_3$ | H | $CH(CH_3)-CH_2CH_3$ | CH | $n_D^{20}$: 1,4796 |
| 62 | $CH_3$ | $OCH_3$ | $OCH_3$ | H | $CH(CH_3)-CH_2CH_3$ | N | $n_D^{20}$: 1,4864 |
| 63 | $CH_2CH_3$ | $OCH_3$ | $OCH_3$ | H | $CH(CH_3)-CH_2CH_3$ | N | $n_D^{20}$: 1,4777 |
| 64 | Tetra-hydro-furfuryl | $OCH_3$ | $OCH_3$ | H | $CH(CH_3)_2$ | N | $n_D^{20}$: 1,4903 |
| 65 | H | $OCH_3$ | $OCH_3$ | H | $CH_3$ | CH | Fp.: 79°C (Zersetzung) |
| 66 | $CH_2CH_3$ | $OCH_3$ | $OCH_3$ | H | $CH_2CH_3$ | CH | $n_D^{20}$: 1,4816 |
| 67 | $CH_2CH_3$ | $OCH_3$ | $OCH_3$ | H | $CH_2CH_3$ | CH | $n_D^{20}$: 1,4807 |
| 68 | H | $OCH_3$ | $OCH_3$ | H | $CH(CH_3)-CH_2CH_3$ | CH | Fp.: 82°C (Zersetzung) |
| 69 | H | $OCH_3$ | $OCH_3$ | H | $CH_2CH_3$ | CH | Fp.: 97°C (Zersetzung) |
| 70 | $N=C(CH_3)_2$ | $OCH_3$ | $OCH_3$ | H | $CH(CH_3)-CH_2CH_3$ | CH | Fp.: 124°C (Zersetzung) |
| 71 | $N=C(CH_3)_2$ | $OCH_3$ | $OCH_3$ | H | $(CH_2)_2CH_3$ | CH | $n_D^{20}$: 1,4974 |

| Beispiel Nr. | $R^1$ | $R^2$ | $R^3$ | $R^4$ | $R^5$ | X | Physikalische Konstante |
|---|---|---|---|---|---|---|---|
| 72 | $N=C(CH_3)-CH_2CH_3$ | $OCH_3$ | $OCH_3$ | H | $CH(CH_3)_2$ | CH | $n_D^{20}$: 1,4946 |
| 73 | Benzyl | $OCH_3$ | $OCH_3$ | H | $CH(CH_3)_2$ | N | $n_D^{20}$: 1,5226 |
| 75 | $(CH_2)_2O-N=C(CH_3)_2$ | $OCH_3$ | $OCH_3$ | H | $CH(CH_3)_2$ | CH | $n_D^{20}$: 1,4852 |
| 76 | $CH_2CH_3$ | $OCH_2CH_3$ | $OCH_2CH_3$ | H | $CH(CH_3)_2$ | N | $n_D^{20}$: 1,4758 |
| 79 | $(CH_2)_2-N(CH_3)_2$ | $OCH_3$ | $OCH_3$ | H | $CH(CH_3)_2$ | N | $n_D^{20}$: 1,4850 |
| 80 | $(CH_2)_2-N(CH_2)_5$ | $OCH_3$ | $OCH_3$ | H | $CH(CH_3)_2$ | N | $n_D^{20}$: 1,4967 |
| 81 | $(CH_2)_3CH_3$ | $OCH_3$ | $OCH_3$ | H | $CH(CH_3)_2$ | N | $n_D^{20}$: 1,4763 |
| 82 | $(CH_2)_2O-CH_2CH_3$ | $OCH_3$ | $OCH_3$ | H | $CH(CH_3)_2$ | N | $n_D^{20}$: 1,4755 |
| 83 | $(CH_2)_2O-(CH_2)_2O-CH_2CH_3$ | $OCH_3$ | $OCH_3$ | H | $CH(CH_3)_2$ | N | $n_D^{20}$: 1,4759 |
| 84 | $(CH_2)_2O-$benzyl | $OCH_3$ | $OCH_3$ | H | $CH(CH_3)_2$ | N | $n_D^{20}$: 1,5166 |

| Beispiel Nr. | $R^1$ | $R^2$ | $R^3$ | $R^4$ | $R^5$ | X | Physikalische Konstante |
|---|---|---|---|---|---|---|---|
| 85 | $(CH_2)_2OH$ | $OCH_3$ | $OCH_3$ | H | $CH(CH_3)_2$ | N | $n_D^{20}$: 1,4972 |
| 86 | $CH_2CO_2H$ | $OCH_3$ | $OCH_3$ | H | $CH(CH_3)_2$ | N | |
| 87 | $CH_2CO_2CH_3$ | $OCH_3$ | $OCH_3$ | H | $CH(CH_3)_2$ | N | |
| 88 | H | $OCH_3$ | $OCH_3$ | H | 1-Methyl-benzyl | CH | Fp.: 141°C (Zersetzung) |
| 89 | $CH_3$ | $OCH_3$ | $OCH_3$ | H | 1-Methyl-benzyl | CH | Fp.: 81°C |
| 90 | $CH_2CH_3$ | $OCH_2CH_3$ | $OCH_3$ | H | $CH(CH_3)_2$ | N | $n_D^{20}$: 1,4732 |
| 91 | $CH_2CO_2CH_3$ | $OCH_3$ | $OCH_3$ | H | $CH(CH_3)_2$ | CH | $n_D^{20}$: 1,4859 |
| 92 | $CH(CH_3)-CO_2CH_3$ | $OCH_3$ | $OCH_3$ | H | $CH(CH_3)_2$ | CH | $n_D^{20}$: 1,4809 |
| 93 | $(CH_2)_2-N(CH_3)_3J$ | $OCH_3$ | $OCH_3$ | H | $CH(CH_3)_2$ | N | Fp.: 74-76°C |
| 94 | $CH_2CH_3$ | $OCH_3$ | $OCH_2CH_3$ | H | $CH(CH_3)_2$ | N | $n_D^{20}$: 1,4746 |
| 96 | $CH_2CH_3$ | $OCH_3$ | $OCH(CH_3)_2$ | H | $CH(CH_3)_2$ | N | $n_D^{20}$: 1,4724 |
| 97 | $CH_2CH_3$ | $OCH(CH_3)_2$ | $OCH(CH_3)_2$ | H | $CH(CH_3)_2$ | N | $n_D^{20}$: 1,4688 |
| 98 | $CH_3$ | $OCH_3$ | $OCH_3$ | H | Benzyl | CH | $n_D^{24}$: 1,5350 |

| Beispiel Nr. | $R^1$ | $R^2$ | $R^3$ | $R^4$ | $R^5$ | X | Physikalische Konstante |
|---|---|---|---|---|---|---|---|
| 100 | H | $OCH_3$ | $OCH_3$ | H | Benzyl | CH | Fp.: 100-104°C |
| 102 | $CH_3$ | $OCH_3$ | $OCH_3$ | H | 3-Chlor-benzyl | CH | $n_D^{24}$: 1,5410 |
| 104 | H | $OCH_3$ | $OCH_3$ | H | 3-Chlor-benzyl | CH | Fp.: 117-118°C |
| 105 | $CH_3$ | $OCH_3$ | $OCH_3$ | H | 4-Chlor-benzyl | CH | Fp.: 66-68°C |
| 106 | H | $OCH_3$ | $OCH_3$ | H | 4-Chlor-benzyl | CH | Fp.: 103-104°C |
| 108 | $CH_3$ | $OCH_3$ | $OCH_3$ | H | $CH_2C{\equiv}CH$ | CH | $n_D^{24}$: 1,5020 |
| 109 | $(CH_2)_3$-$CO_2CH_2CH_3$ | $OCH_3$ | $OCH_3$ | H | $CH(CH_3)_2$ | CH | $n_D^{20}$: 1,4768 |

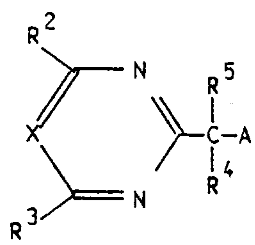

| Beispiel Nr. | A | $R^2$ | $R^3$ | $R^4$ | $R^5$ | X | Physikalische Konstante |
|---|---|---|---|---|---|---|---|
| 110 | CN | $OCH_3$ | $OCH_3$ | H | $CH(CH_3)_2$ | CH | $n_D^{20}$: 1,4930 |
| 111 | $CONH_2$ | $OCH_3$ | $OCH_3$ | H | $(CH_2)_3CH_3$ | CH | Fp.: 75-76°C |
| 112 | $CONH_2$ | $OCH_3$ | $OCH_3$ | H | $CH(CH_3)_2$ | CH | Fp.: 103-105°C |
| 113 | $CON(CH_3)_2$ | $OCH_3$ | $OCH_3$ | H | $CH(CH_3)_2$ | CH | $n_D^{20}$: 1,5030 |
| 114 | $CON(C_2H_5)_2$ | $OCH_3$ | $OCH_3$ | H | $CH(CH_3)_2$ | CH | $n_D^{20}$: 1,4960 |

Die folgenden Beispiele erläutern die Anwendungsmöglichkeiten der erfindungsgemäßen Verbindungen:

**Beispiel A**

Im Gewächshaus wurden die aufgeführten Pflanzenspezies vor dem Auflaufen mit den aufgeführten Verbindungen in einer Aufwandmenge von 0,3 kg Wirkstoff/ha behandelt. Die Verbindungen wurden zu diesem Zweck als Emulsionen oder Suspensionen mit 500 Litern Wasser/ha gleichmäßig über den Boden versprüht. Hier zeigten drei Wochen nach der Behandlung die erfindungsgemäßen Verbindungen eine ausgezeichnete Wirkung gegen das Unkraut. Das Vergleichsmittel zeigte nicht die gleichhohe Wirksamkeit.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
AGRRE = Elymus repens
BROTE = Bromus tectorum
SORHA = Sorghum halepense
ABUTH = Abutilon theophrasti
IPOSS = Ipomoea purpurea
SEBEX = Sesbania exaltata

| Erfindungsgemäße Verbindungen | AGRRE | BROTE | SORHA | ABUTH | IPOSS | SEBEX |
|---|---|---|---|---|---|---|
| Beispiel 5 | 3 | 4 | 3 | 3 | 3 | 3 |
| Beispiel 15 | 3 | 4 | 3 | 3 | 3 | 3 |
| Beispiel 23 | 3 | 4 | 3 | - | 3 | 3 |
| Beispiel 33 | 3 | 3 | 3 | 2 | 2 | 2 |
| Beispiel 36 | 3 | 4 | 4 | 3 | 2 | 3 |
| Beispiel 62 | 3 | 4 | 3 | - | - | 2 |
| Beispiel 63 | 3 | 3 | 3 | 3 | - | 3 |
| Beispiel 64 | 3 | 3 | 3 | 3 | 2 | 3 |
| Beispiel 70 | 3 | 4 | 3 | 4 | 3 | 3 |
| Beispiel 72 | 3 | 4 | 3 | 3 | 2 | 3 |
| Beispiel 73 | 3 | 4 | 3 | 4 | 2 | 3 |
| Unbehandelt | 0 | 0 | 0 | 0 | 0 | 0 |
| Vergleichsmittel | | | | | | |
| Imazamethabenz | 1 | 2 | 2 | 2 | 2 | 1 |

**Beispiel B**

Im Gewächshaus wurden die aufgeführten Pflanzenspezies nach dem Auflaufen mit den aufgeführten Verbindungen in einer Aufwandmenge von 0,1 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigte zwei Wochen nach der Behandlung die erfindungsgemäße Verbindung eine hohe Kulturpflanzenselektivität in Sojabohne,

Baumwolle, Sonnenblume und Weizen bei ausgezeichneter Wirkung gegen das Unkraut. Das Vergleichsmittel zeigte nicht die gleichhohe Wirksamkeit.

In der folgenden Tabelle bedeuten:

| | |
|---|---|
| 0 = | keine Schädigung |
| 1 = | 1 - 24 % Schädigung |
| 2 = | 25 - 74 % Schädigung |
| 3 = | 75 - 89 % Schädigung |
| 4 = | 90 - 100 % Schädigung |
| GLXMA = | Glycine maxima |
| GOSHI = | Gossypium hirsutum |
| HELAN = | Helianthus annuus |
| TRZAX = | Triticum aestivum |
| BROTE = | Bromus tectorum |
| GALAP = | Galium aparine |
| VERPE = | Veronica persica |
| VIOSS = | Viola sp. |

| Erfindungsgemäße Verbindung | GLXMA | GOSHI | HELAN | TRZAX | BROTE | GALAP | VERPE | VIOSS |
|---|---|---|---|---|---|---|---|---|
| Beispiel 5 | 0 | 0 | 0 | 1 | 3 | 3 | 3 | 3 |
| Unbehandelt | 0 | 0 | 0 | 0 | 0 | 0 | 0 | 0 |
| Vergleichsmittel | | | | | | | | |
| Imazamethabenz | 0 | 1 | 1 | 0 | 0 | 1 | 2 | 1 |

**Beispiel C**

Im Gewächshaus wurden die aufgeführten Pflanzenspezies vor dem Auflaufen mit den aufgeführten Verbindungen in einer Aufwandmenge von 0,3 kg Wirkstoff/ha behandelt. Die Verbindungen wurden zu diesem Zweck als Emulsionen oder Suspensionen mit 500 Litern Wasser/ha gleichmäßig über den Boden versprüht. Hier zeigten drei Wochen nach der Behandlung die erfindungsgemäßen Verbindungen eine hohe Kulturpflanzenselektivität in Sojabohne, Baumwolle und Sonnenblume bei ausgezeichneter Wirkung gegen das Unkraut. Das Vergleichsmittel zeigte nicht die gleichhohe Wirksamkeit.

In der folgenden Tabelle bedeuten:

| | |
|---|---|
| 0 = | keine Schädigung |
| 1 = | 1 - 24 % Schädigung |
| 2 = | 25 - 74 % Schädigung |
| 3 = | 75 - 89 % Schädigung |
| 4 = | 90 - 100 % Schädigung |
| GLXMA = | Glycine maxima |
| GOSHI = | Gossypium hirsutum |
| HELAN = | Helianthus annuus |
| BROTE = | Bromus tectorum |
| PANSS = | Panicum maximum |

| Erfindungsgemäße Verbindungen | GLXMA | GOSHI | HELAN | BROTE | PANSS |
|---|---|---|---|---|---|
| Beispiel 4 | 0 | 0 | 0 | 3 | 3 |
| Beispiel 8 | 1 | 1 | 1 | 3 | 3 |
| Beispiel 20 | 1 | - | 0 | 3 | 3 |
| Beispiel 31 | 0 | 0 | 1 | 3 | 3 |
| Beispiel 32 | 0 | 1 | 1 | - | 3 |
| Beispiel 34 | 0 | - | 1 | 3 | 3 |
| Beispiel 35 | 0 | - | 0 | 3 | 3 |
| Beispiel 37 | 0 | 0 | 0 | 3 | 3 |
| Beispiel 38 | - | - | 1 | 3 | 3 |
| Beispiel 60 | 1 | 0 | 0 | 3 | 3 |
| Beispiel 61 | 0 | 0 | 0 | 3 | 3 |
| Beispiel 68 | 1 | 1 | 1 | - | 3 |
| Beispiel 71 | - | 1 | 1 | 3 | 3 |
| Beispiel 75 | 0 | - | 1 | 3 | 3 |
| Unbehandelt | 0 | 0 | 0 | 0 | 0 |
| Vergleichsmittel | | | | | |
| Imazamethabenz | 1 | 0 | 0 | 0 | 1 |

**Beispiel D**

Im Gewächshaus wurden die aufgeführten Pflanzenspezies vor dem Auflaufen mit den aufgeführten Verbindungen in einer Aufwandmenge von 0,1 kg Wirkstoff/ha behandelt. Die Verbindungen wurden zu diesem Zweck als Emulsionen oder Suspensionen mit 500 Litern Wasser/ha gleichmäßig über den Boden versprüht. Hier zeigte drei Wochen nach der Behandlung die erfindungsgemäße Verbindung eine hohe Kulturpflanzenselektivität in Weizen bei ausgezeichneter Wirkung gegen das Unkraut. Das Vergleichsmittel zeigte nicht die gleichhohe Wirksamkeit.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
TRZAX = Triticum aestivum
VERPE = Veronica persica
VIOSS = Viola sp.

| Erfindungsgemäße Verbindung | TRZAX | VERPE | VIOSS |
|---|---|---|---|
| Beispiel 22 | 0 | 3 | 3 |
| Unbehandelt | 0 | 0 | 0 |
| Vergleichsmittel | | | |
| Imazamethabenz | 0 | 1 | 2 |

**Beispiel E**

Im Gewächshaus wurden die aufgeführten Pflanzenspezies vor dem Auflaufen mit den aufgeführten Verbindungen in einer Aufwandmenge von 1,0 kg Wirkstoff/ha behandelt. Die Verbindungen wurden zu diesem Zweck als Emulsionen oder Suspensionen mit 500 Litern Wasser/ha gleichmäßig über den Boden versprüht. Hier zeigten drei Wochen nach der Behandlung die erfindungsgemäßen Verbindungen eine hohe Kulturpflanzenselektivität in Sojabohne, Baumwolle und Mais bei ausgezeichneter Wirkung gegen das Unkraut. Das Vergleichsmittel zeigte nicht die gleichhohe Selektivität.

In der folgenden Tabelle bedeuten:

| | |
|---|---|
| 0 = | keine Schädigung |
| 1 = | 1 - 24 % Schädigung |
| 2 = | 25 - 74 % Schädigung |
| 3 = | 75 - 89 % Schädigung |
| 4 = | 90 - 100 % Schädigung |
| GLXMA = | Glycine maxima |
| GOSHI = | Gossypium hirsutum |
| ZEAMX = | Zea mays |
| BROTE = | Bromus tectorum |
| SETVI = | Setaria viridis |
| GALAP = | Galium aparine |
| MATCH = | Matricaria chamomilla |
| VERPE = | Veronica persica |
| VIOSS = | Viola sp. |

| Erfindungsgemäße Verbindungen | GLXMA | GOSHI | ZEAMX | BROTE | SETVI | GALAP | MATCH | VERPE | VIOSS |
|---|---|---|---|---|---|---|---|---|---|
| Beispiel 24 | 0 | 0 | 0 | 3 | 3 | 3 | 3 | 3 | 3 |
| Beispiel 59 | 0 | 1 | - | - | 3 | 3 | - | 3 | 3 |
| Beispiel 67 | 1 | 0 | 1 | 3 | - | 3 | - | 3 | 3 |
| Unbehandelt | 0 | 0 | 0 | 0 | 0 | 0 | 0 | 0 | 0 |
| Vergleichsmittel | | | | | | | | | |
| Imazamethabenz | 3 | 2 | 2 | 2 | 3 | 3 | 3 | 3 | 3 |

## Patentansprüche

1. Substituierte 2-Pyrimidinyl- und 2-Triazinylessigsäurederivate der allgemeinen Formel I

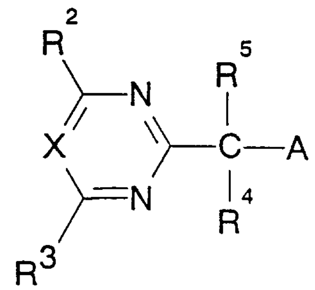

(I),

in der

A die Gruppe -CO-OR[1],

$R^1$ ein Wasserstoffatom, einen $C_1$-$C_{12}$-Alkylrest, einen durch Hydroxy, Halogen, $C_1$-$C_4$-Alkoxy, $C_1$-$C_4$-Alkylthio, $C_3$-$C_6$-Cycloalkyl, Benzyl, Furyl, Tetrahydrofuryl, Phenyl, Halogenphenyl, $C_1$-$C_4$-Alkylphenyl, $C_1$-$C_4$-Alkoxyphenyl, Nitrophenyl, Cyano, Carboxyl, $C_1$-$C_4$-Alkoxycarbonyl, $C_1$-$C_4$-Alkylamino, Di-$C_1$-$C_4$-alkylamino, Tri-$C_1$-$C_4$-alkylammoniumhalogenid, $C_2$-$C_5$-Alkylenimino oder Di-$C_1$-$C_4$-alkylmethyleniminooxy substituierten $C_1$-$C_{12}$-Alkylrest, einen durch ein oder mehrere Sauerstoffatome oder Schwefelatome unterbrochenen $C_2$-$C_{12}$-Alkylrest, einen durch ein oder mehrere Sauerstoffatome oder Schwefelatome unterbrochenen und durch Hydroxy, Halogen, $C_1$-$C_4$-Alkoxy, $C_1$-$C_4$-Alkylthio, $C_3$-$C_6$-Cycloalkyl, Benzyl, Furyl, Tetrahydrofuryl, Phenyl, Halogenphenyl, $C_1$-$C_4$-Alkylphenyl, $C_1$-$C_4$-Alkoxyphenyl, Nitrophenyl, Cyano, Carboxyl, $C_1$-$C_4$-Alkoxycarbonyl, $C_1$-$C_4$-Alkylamino oder Di-$C_1$-$C_4$-alkylamino substituierten $C_2$-$C_{12}$-Alkylrest, einen $C_3$-$C_8$-Alkenylrest, einen durch $C_1$-$C_3$-Alkoxy, Phenyl oder Halogen substituierten $C_3$-$C_8$-Alkenylrest, einen $C_3$-$C_8$-Alkinylrest, einen durch $C_1$-$C_3$-Alkoxy, Phenyl oder Halogen substituierten $C_3$-$C_8$-Alkinylrest, einen $C_3$-$C_6$-Cycloalkylrest, einen durch Methyl substituierten $C_3$-$C_6$-Cycloalkylrest oder einen Di-$C_1$-$C_4$-Alkylmethyleniminorest oder einen $C_3$-$C_6$-Cycloalkyleniminorest, mit der Maßgabe, daß $R^1$ nicht der Ethylrest ist, wenn $R^5$ der Benzylrest ist,

$R^2$ einen $C_1$-$C_4$-Alkoxyrest,

$R^3$ einen $C_1$-$C_4$-Alkoxyrest,

$R^4$ ein Wasserstoffatom,

$R^5$ einen $C_1$-$C_{10}$-Alkylrest, einen ein- oder mehrfach, gleich oder verschieden durch $C_1$-$C_4$-Alkoxy, $C_1$-$C_4$-Alkylthio, Amino, $C_1$-$C_4$-Alkylamino, Di-$C_1$-$C_4$-alkylamino, Nitro, Halogen, Phenyl oder Halogenphenyl substituierten $C_1$-$C_{10}$-Alkylrest, einen $C_2$-$C_{10}$-Alkenylrest, einen ein- oder mehrfach, gleich oder verschieden durch $C_1$-$C_4$-Alkoxy, $C_1$-$C_4$-Alkylthio, Amino, $C_1$-$C_4$-Alkylamino, Di-$C_1$-$C_4$-alkylamino, Nitro, Halogen oder Phenyl substituierten $C_2$-$C_{10}$-Alkenylrest, einen $C_2$-$C_{10}$-Alkinylrest, einen ein- oder mehrfach, gleich oder verschieden durch $C_1$-$C_4$-Alkoxy, $C_1$-$C_4$-Alkylthio, Amino, $C_1$-$C_4$-Alkylamino, Di-$C_1$-$C_4$-alkylamino, Nitro, Halogen oder Phenyl substituierten $C_2$-$C_{10}$-Alkinylrest, einen $C_3$-$C_8$-Cycloalkylrest, einen ein- oder mehrfach, gleich oder verschieden durch $C_1$-$C_4$-Alkyl, $C_1$-$C_4$-Alkoxy, $C_1$-$C_4$-Alkylthio, Amino, $C_1$-$C_4$-Alkylamino, Di-$C_1$-$C_4$-alkylamino, Nitro, Trifluormethyl, Halogen oder Phenyl substituierten $C_3$-$C_8$-Cycloalkylrest, einen $C_4$-$C_8$-Cycloalkenylrest oder einen ein- oder mehrfach, gleich oder verschieden durch $C_1$-$C_4$-Alkyl, $C_1$-$C_4$-Alkoxy, $C_1$-$C_4$-Alkylthio, Amino, $C_1$-$C_4$-Alkylamino, Di-$C_1$-$C_4$-alkylamino, Nitro, Trifluormethyl, Halogen oder Phenyl substituierten $C_4$-$C_8$-Cycloalkenylrest und

X eine Methingruppe oder ein Stickstoffatom,

bedeuten, sowie deren Alkalimetallsalze, Erdalkalimetallsalze, Ammoniumsalze und organisch substituierten Ammoniumsalze und deren optisch aktive Isomeren.

2. Verfahren zur Herstellung von Verbindungen der allgemeinen Formel I, dadurch gekennzeichnet, daß man

A) Verbindungen der allgemeinen Formel II

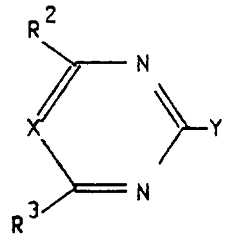

(II) ,

in der $R^2$, $R^3$ und X die unter der allgemeinen Formel I genannten Bedeutungen haben und Y für ein Halogenatom, eine Alkylsulfonylgruppe oder eine Phenylsulfonylgruppe steht, mit Verbindungen der allgemeinen Formel III

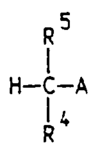

(III) ,

in der A, $R^4$ und $R^5$ die unter der allgemeinen Formel I genannten Bedeu tungen haben, in einem geeigneten Lösungsmittel in Gegenwart einer geeigneten Base umsetzt, oder

B) Verbindungen der allgemeinen Formel IV

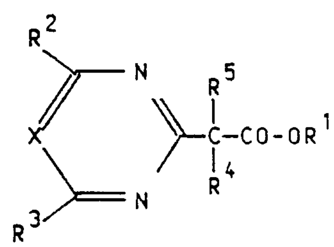

(IV) ,

in der $R^1$, $R^2$, $R^3$, $R^4$, $R^5$ und X die unter der allgemeinen Formel I angegebenen Bedeutungen haben, $R^1$ aber nicht für ein Wasserstoffatom steht, in einem geeigneten polaren Lösungsmittel mit einer Alkalimetallbase oder einer Erdalkalimetallbase zu Verbindungen der allgemeinen Formel V

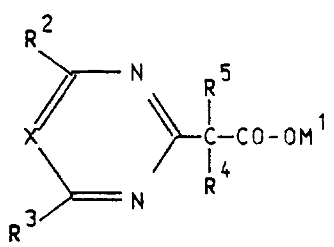

(V) ,

in der $R^2$, $R^3$, $R^4$, $R^5$ und X die unter der allgemeinen Formel I angegebenen Bedeutungen haben und $M^1$ für ein Alkalimetallatom oder ein Äquivalent eines Erdalkalimetallatoms steht, umsetzt, oder

C) Verbindungen der allgemeinen Formel V

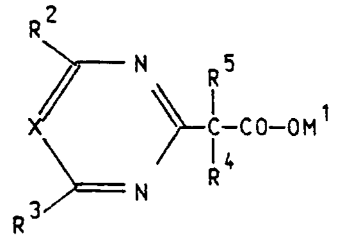

(V) ,

in der $R^2$, $R^3$, $R^4$, $R^5$, $M^1$ und X die unter der allgemeinen Formel V angegebenen Bedeutungen haben, mit einer geeigneten Säure in einem geeigneten Lösungsmittel zu Verbindungen der allgemeinen Formel VI

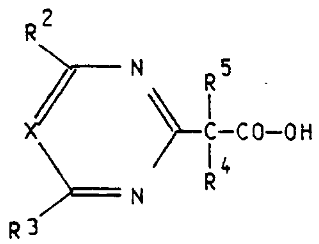

(VI) ,

in der $R^2$, $R^3$, $R^4$, $R^5$ und X die unter der allgemeinen Formel I angegebenen Bedeutungen haben, umsetzt, oder

D) Verbindungen der allgemeinen Formel VI

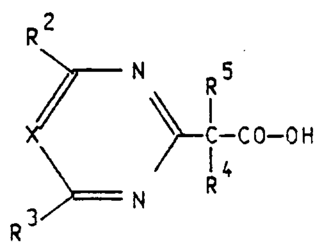

(VI) ,

in der $R^2$, $R^3$, $R^4$, $R^5$ und X die unter der allgemeinen Formel I angegebenen Bedeutungen haben, mit einer geeigneten Base in einem geeigneten Lösungsmittel zu einer Verbindung der allgemeinen Formel VII

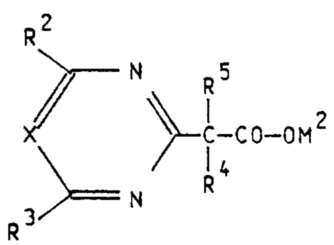

(VII) ,

in der $R^2$, $R^3$, $R^4$, $R^5$ und X die unter der allgemeinen Formel I angegeben Bedeutungen haben und $M^2$ für ein Alkalimetallatom, ein Äquivalent eines Erdalkalimetallatom, ein Ammoniumion oder eine organisch substituierte Ammoniumgruppe steht, umsetzt, oder

E) eine Verbindung der allgemeinen Formel VIII

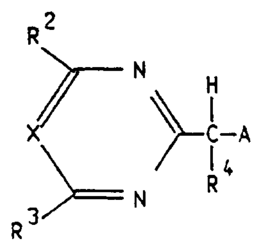

(VIII) ,

in der A, $R^2$, $R^3$, $R^4$ und X die unter der allgemeinen Formel I angegebenen Bedeutungen haben, mit einer Verbindung der allgemeinen Formel IX

$$R^5 - Z \qquad (IX),$$

in der $R^5$ die unter der allgemeinen Formel I angegebenen Bedeutungen hat und Z für ein Halogenatom, eine Alkylsulfonyloxygruppe, eine Phenylsulfonyloxygruppe oder eine Gruppe -O-$SO_2$-O-Alkyl steht, in einem geeigneten Lösungsmittel in Anwesenheit einer geeigneten Base umsetzt.

**3.** Mittel mit herbizider, fungizider und pflanzenwachstumsregulierender Wirkung, gekennzeichnet durch einen Gehalt an mindestens einer Verbindung gemäß dem Anspruch 1.

**4.** Verwendung von Mitteln gemäß dem Anspruch 3 zur Bekämpfung monokotyler und dikotyler Unkrautarten in landwirtschaftlichen Hauptkulturen.

**5.** Verwendung von Mitteln gemäß dem Anspruch 3 zur Bekämpfung eines Pilzbefalles in landwirtschaftlichen Kulturen.

**6.** Verwendung von Mitteln gemäß Anspruch 3 zur Wachstumsbeeinflussung von Kulturpflanzen.

**7.** Verfahren zur Herstellung von Mitteln mit herbizider, fungizider und wachstumsregulierender Wirkung, dadurch gekennzeichnet, daß man Verbindungen der allgemeinen Formel I gemäß dem Anspruch 1 mit Träger- und/oder Hilfsstoffen vermischt.

**8.** 2-(4,6-Dimethoxy-pyrimidin-2-yl)-3-cyclopropyl-propansäuremethylester.

## Claims

**1.** Substituted 2-pyrimidinyl- and 2-triazinylacetic acid derivatives of general formula I

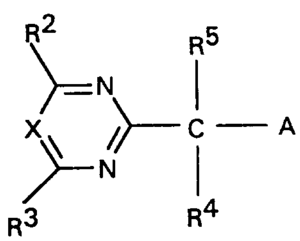

(I)

in which

A is the group -CO-$OR^1$,

$R^1$ is a hydrogen atom, a $C_1$-$C_{12}$-alkyl group, a $C_1$-$C_{12}$-alkyl group, substituted by hydroxy, halogen, $C_1$-$C_4$-alkoxy, $C_1$-$C_4$-alkylthio, $C_3$-$C_6$-cycloalkyl, benzyl, furyl, tetrahydrofuryl, phenyl, halophenyl, $C_1$-$C_4$-alkylphenyl, $C_1$-$C_4$-alkoxyphenyl, nitrophenyl, cyano, carboxy, $C_1$-$C_4$-alkoxycarbonyl, $C_1$-$C_4$-alkylamino, di-$C_1$-$C_4$-alkylamino, tri-$C_1$-$C_4$-alkylammoniumhalide, $C_2$-$C_5$-alkylenimino or di-$C_1$-$C_4$-alkylmethyleniminooxy, $C_2$-$C_{12}$-alkyl, interrupted by one or more oxygen or sulfur atoms, $C_2$-$C_{12}$-alkyl, interrupted by one or more oxygen or sulfur atoms and optionally substituted by hydroxy, halogen, $C_1$-$C_4$-alkoxy, $C_1$-$C_4$-

alkylthio, $C_3$-$C_6$-cycloalkyl, benzyl, furyl, tetrahydrofuryl, phenyl, halophenyl, $C_1$-$C_4$-alkylphenyl, $C_1$-$C_4$-alkoxyphenyl, nitrophenyl, cyano, carboxy, $C_1$-$C_4$-alkoxycarbonyl, $C_1$-$C_4$-alkylamino or di-$C_1$-$C_4$-alkylamino, a $C_3$-$C_8$-alkenyl group, a $C_3$-$C_8$-alkenyl group substituted by $C_1$-$C_3$-alkoxy, phenyl or halogen, a $C_3$-$C_8$-alkynyl group, a $C_3$-$C_8$-alkynyl group substituted by $C_1$-$C_3$-alkoxy, phenyl or halogen, a $C_3$-$C_8$-cycloalkyl group, a $C_3$-$C_8$-cycloalkyl group substituted by methyl, a di-$C_1$-$C_4$-alkylmethylenimino group or a $C_3$-$C_6$-cycloalkylenimino group; with the proviso that $R^1$ is not ethyl, when $R^5$ is benzyl, $R^2$ is a $C_1$-$C_4$-alkyl group,

$R^3$ is a $C_1$-$C_4$-alkyl group,

$R^4$ is hydrogen,

$R^5$ is a $C_1$-$C_{10}$-alkyl group, a $C_1$-$C_{10}$-alkyl group substituted by one or more of the same or different $C_1$-$C_4$-alkoxy, $C_1$-$C_4$-alkylthio, amino, $C_1$-$C_4$-alkylamino, di-$C_1$-$C_4$-alkylamino, nitro, halogen, phenyl or halophenyl, a $C_2$-$C_{10}$-alkenyl group, a $C_2$-$C_{10}$-alkenyl group substituted by one or more of the same or different $C_1$-$C_4$-alkoxy, $C_1$-$C_4$-alkylthio, amino, $C_1$-$C_4$-alkylamino, di-$C_1$-$C_4$-alkylamino, nitro, halogen or phenyl, a $C_2$-$C_{10}$-alkynyl group, a $C_2$-$C_{10}$-alkynyl group optionally substituted by one or more of the same or different $C_1$-$C_4$-alkoxy, $C_1$-$C_4$-alkylthio, amino, $C_1$-$C_4$-alkylamino, di-$C_1$-$C_4$-alkylamino, nitro, halogen or phenyl, a $C_3$-$C_8$-cycloalkyl group, a $C_3$-$C_8$-cycloalkyl substituted by one or more of the same or different $C_1$-$C_4$-alkyl, $C_1$-$C_4$-alkoxy, $C_1$-$C_4$-alkylthio, amino, $C_1$-$C_4$-alkylamino, di-$C_1$-$C_4$-alkylamino, nitro, trifluoromethyl, halogen or phenyl, a $C_4$-$C_8$-cycloalkenyl group, a $C_4$-$C_8$-cycloalkenyl group optionally substituted by one or more of the same or different $C_1$-$C_4$-alkyl, $C_1$-$C_4$-alkoxy, $C_1$-$C_4$-alkylthio, amino, $C_1$-$C_4$-alkylamino, di-$C_1$-$C_4$-alkylamino, nitro, trifluoromethyl, halogen or phenyl, and

X is a methine group or nitrogen,

as well as their alkali metal, alkaline earth metal, ammonium and organic ammonium salts, and their optically active isomers.

**2.** Process for the preparation of compounds of general formula I characterised in that

A) compounds of general formula II

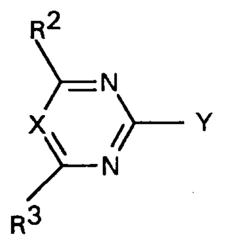

(II)

in which $R^2$, $R^3$ and X have the meanings given under general formula I, and Y is a halogen atom, an alkylsulfonyl group or a phenylsulfonyl group, are reacted with a compound of general formula III

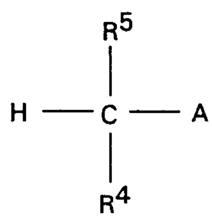

(III)

in which A, $R^4$ and $R^5$ have the meanings given under general formula I, in a suitable solvent in the presence of a suitable base, or

B) compounds of general formula IV

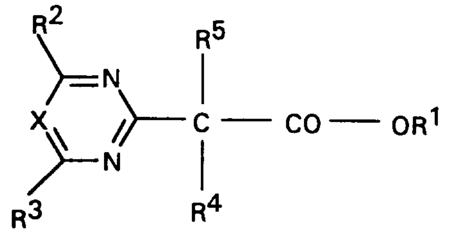

(IV)

in which $R^1$, $R^2$, $R^3$, $R^4$, $R^5$ and X have the meanings given under general formula I, except for $R^1$ being hydrogen, are treated with an alkali metal base or alkaline earth metal base, in a suitable polar solvent, to give compounds of general formula V

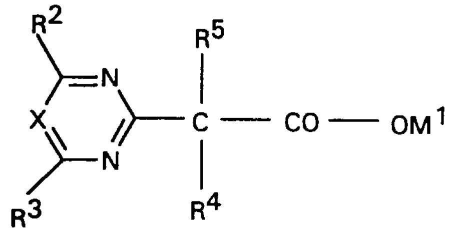

(V)

in which $R^2$, $R^3$, $R^4$, $R^5$ and X have the meanings given under general formula I and $M^1$ is an alkali metal atom or an equivalent of an alkaline earth metal atom, or

C) compounds of general formula V

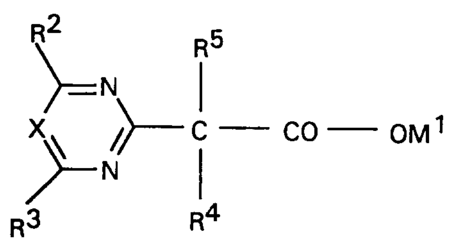

(V)

in which $R^2$, $R^3$, $R^4$, $R^5$, $M^1$ and X have the meanings given under general formula V, are treated with a suitable acid, in a suitable solvent, to give compounds of general formula VI

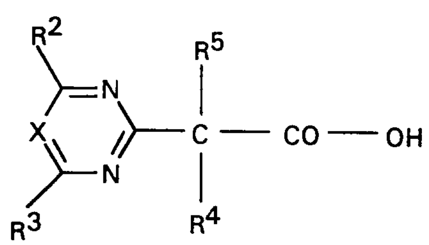

(VI)

in which $R^2$, $R^3$, $R^4$, $R^5$ and X have the meanings given under general formula I, or

D) compounds of general formula VI

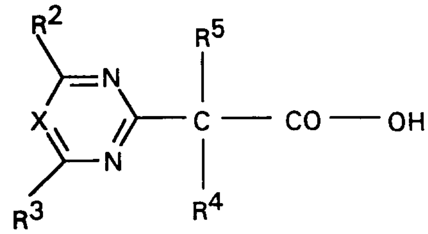

(VI)

in which $R^2$, $R^3$, $R^4$, $R^5$ and X have the meanings given under general formula I, are treated with a suitable base, in a suitable solvent, to give compounds of general formula VII

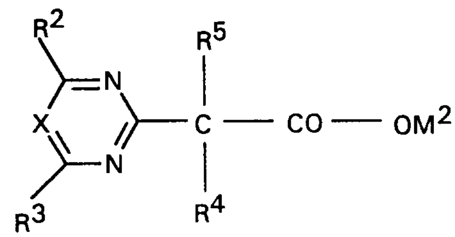

(VII)

in which $R^2$, $R^3$, $R^4$, $R^5$ and X have the meanings given under general formula I and $M^2$ is an alkali metal atom or an equivalent of an alkaline earth metal atom, an ammonium ion or an organic ammonium group, or

E) a compound of general formula VIII

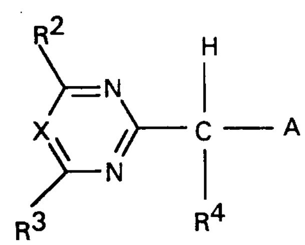

(VIII)

in which A, $R^2$, $R^3$, $R^4$ and X have the meanings given under general formula I, is reacted with a compound of general formula IX

$$R^5\text{-}Z \qquad (IX)$$

in which $R^5$ has the meaning given under general formula I, and Z is a halogen atom, an alkylsulfonyl group, a phenylsulfonyl group or a group $-O-SO_2-O$-alkyl, in a suitable solvent, in the presence of a suitable base.

**3.** Herbicidal, fungicidal and plant growth regulant composition characterised by a content of at least one compound according to claim 1.

**4.** Use of compositions according to claim 3 for the control of monocotyledonous and dicotyledonous weeds in crops.

**5.** Use of compositions according to claim 3 for the control of fungal attack in crops.

**6.** Use of compositions according to claim 3 for the plant growth regulation of crops.

**7.** Process for the preparation of herbicidal, fungicidal and plant growth regulant compositions characterised in that a compound of general formula I according to claim 1 is mixed with carriers and or adjuvants.

**8.** Methyl 2-(4,6-dimethoxypyrimidin-2-yl)-3-cyclopropylpropanoate.

**Revendications**

1. Dérivés de 2-pyrimidinyl- et 2-triazinylacétates de formule générale I

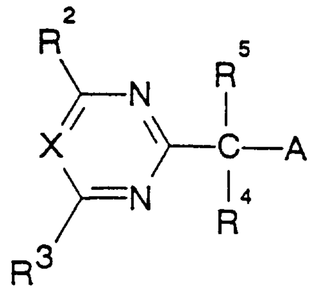

(I),

dans laquelle

A représente le groupe -CO-OR[1],

R1 représente un atome d'hydrogène, un radical alkyle en $C_1$-$C_{12}$, un radical alkyle en $C_1$-$C_{12}$ substitué par hydroxyle, halogéno, alcoxy en $C_1$-$C_4$, (alkyl en $C_1$-$C_4$)-thio, cycloalkyle en $C_3$-$C_6$, benzyle, furyle, tétrahydrofuryle, phényle, halogénophényle, (alkyl en $C_1$-$C_4$)-phényle, (alcoxy en $C_1$-$C_4$)-phényle, nitrophényle, cyano, carboxyle, (alcoxy en $C_1$-$C_4$)-carbonyle, (alkyl en $C_1$-$C_4$)-amino, di-(alkyl en $C_1$-$C_4$)-amino, tri-(alkyl en $C_1$-$C_4$)-ammonium-halogénure, (alkylène en $C_2$-$C_5$)-imino ou di-(alkyl en $C_1$-$C_4$)-méthyléniminooxy, un radical alkyle en $C_2$-$C_{12}$ interrompu par un ou plusieurs atomes d'oxygène ou atomes de soufre, un radical alkyle en $C_2$-$C_{12}$ interrompu par un ou plusieurs atomes d'oxygène et atomes de soufre et substitué par hydroxy, halogéno, alcoxy en $C_1$-$C_4$, (alkyl en $C_1$-$C_4$)-thio, cycloalkyle en $C_3$-$C_6$, benzyle, furyle, tétrahydrofuryle, phényle, halogénophényle, (alkyl en $C_1$-$C_4$)-phényle, (alcoxy en $C_1$-$C_4$)-phényle, nitrophényle, cyano, carboxyle, (alcoxy en $C_1$-$C_4$)-carbonyle, (alkyl en $C_1$-$C_4$)-amino ou di-(alkyl en $C_1$-$C_4$)-amino, un radical alcényle en $C_3$-$C_8$, un radical alcényle en $C_3$-$C_8$ substitué par alcoxy en $C_1$-$C_3$, phényle ou halogéno, un radical alcynyle en $C_3$-$C_8$, un radical alcynyle en $C_3$-$C_8$ substitué par alcoxy en $C_1$-$C_3$, phényle ou halogéno, un radical cycloalkyle en $C_3$-$C_6$, un radical cycloalkyle en $C_3$-$C_6$ substitué par méthyle ou un radical di-(alkyl en $C_1$-$C_4$)-méthylénimino ou un radical (cycloalkyl en $C_3$-$C_6$)-imino, à la condition que $R^1$ ne soit pas le radical éthyle lorsque $R^5$ est le radical benzyle,

$R^2$ un radical alcoxy en $C_1$-$C_4$,

$R^3$ un radical alcoxy en $C_1$-$C_4$,

$R^4$ un atome d'hydrogène,

$R^5$ un radical alkyle en $C_1$-$C_{10}$, un radical alkyle en $C_1$-$C_{10}$ substitué une ou plusieurs fois par des substituants identiques ou différents, par alcoxy en $C_1$-$C_4$, (alkyl en $C_1$-$C_4$)-thio, amino, (alkyl en $C_1$-$C_4$)-amino, di-(alkyl en $C_1$-$C_4$)-amino, nitro, halogéno, phényle ou halogénophényle, un radical alcényle en $C_2$-$C_{10}$, un radical alcényle en $C_2$-$C_{10}$ substitué une ou plusieurs fois par des substituants identiques ou différents, par alcoxy en $C_1$-$C_4$, (alkyl en $C_1$-$C_4$)-thio, amino, (alkyl en $C_1$-$C_4$)-amino, di-(alkyl en $C_1$-$C_4$)-amino, nitro, halogéno ou phényle, un radical alcynyle en $C_2$-$C_{10}$, un radical alcynyle en $C_2$-$C_{10}$ substitué une ou plusieurs fois par des substituants identiques ou différents, par alcoxy en $C_1$-$C_4$, (alkyl en $C_1$-$C_4$)-thio, amino, (alkyl en $C_1$-$C_4$)-amino, di-(alkyl en $C_1$-$C_4$)-amino, nitro, halogéno ou phényle, un radical cycloalkyle en $C_3$-$C_8$, un radical cycloalkyle en $C_3$-$C_8$ substitué une ou plusieurs fois par des substituants identiques ou différents, par alkyle en $C_1$-$C_4$, alcoxy en $C_1$-$C_4$, (alkyl en $C_1$-$C_4$)-thio, amino, (alkyl en $C_1$-$C_4$)-amino, di-(alkyl en $C_1$-$C_4$)-amino, nitro, trifluorométhyle, halogéno ou phényle, un radical cycloalcényle en $C_4$-$C_8$ ou un radical cycloalcényle en $C_4$-$C_8$ substitué une ou plusieurs fois par des substituants identiques ou différents, par alkyle en $C_1$-$C_4$, alcoxy en $C_1$-$C_4$, (alkyl en $C_1$-$C_4$)-thio, amino, (alkyl en $C_1$-$C_4$)-amino, di-(alkyl en $C_1$-$C_4$)-amino, nitro, trifluoro-méthyle, halogéno ou phényle, et

X un groupe méthyne ou un atome d'azote,

ainsi que des sels de métaux alcalins, des sels de métaux alcalino-terreux, des sels d'ammonium et des sels d'ammonium substitués par des radicaux organiques et leurs isomères optiquement actifs.

2. Procédé pour la préparation de composés de formule générale I, caractérisé en ce que

A) on fait réagir des composés de formule générale II

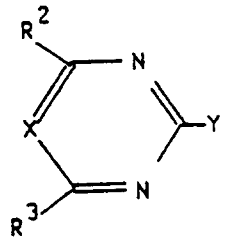

(II)

dans laquelle $R^2$, $R^3$ et X ont les significxations données pour la formule générale I et Y représente un atome d'halogène, un groupe alkylsulfonyle ou un groupe phénylsulfonyle, avec des composés de formule générale III

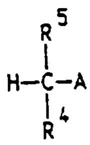

(III)

dans laquelle A, $R^4$ et $R^5$ ont les significations données pour la formule générale I, dans un solvant approprié, en présence d'une base appropriée, ou

B) on fait réagir des composés de formule générale IV

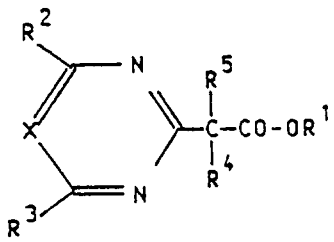

(IV)

dans laquelle $R^1$, $R^2$, $R^3$, $R^4$, $R^5$ et X ont les significations données pour la formule générale I, mais $R^1$ n'est pas un atome d'hydrogène, dans un solvant polaire approprié, avec une base de métal alcalin ou une base de métal alcalino-terreux, pour obtenir des composés de formule générale V

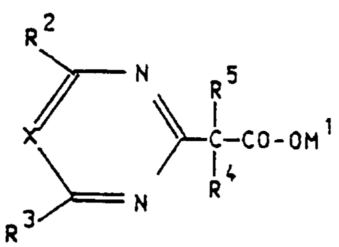

(V)

dans laquelle $R^2$, $R^3$, $R^4$, $R^5$ et X ont les significations données pour la formule générale I et $M^1$ représente un métal alcalin ou un métal alcalino-terreux, ou

C) on fait réagir les composés de formule générale V

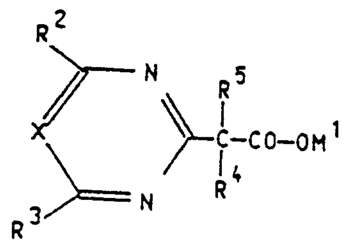

(V)

dans laquelle $R^2$, $R^3$, $R^4$, $R^5$, $M^1$ et X ont les significations données pour la formule générale V, avec un acide apoproprié, dans un solvant approprié, pour obtenir des composés de formule générale VI

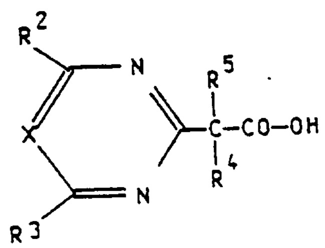

(VI)

dans laquelle $R^2$, $R^3$, $R^4$, $R^5$ et X ont les significations données pour la formule générale I, ou

D) on fait réagir des composés de formule générale VI

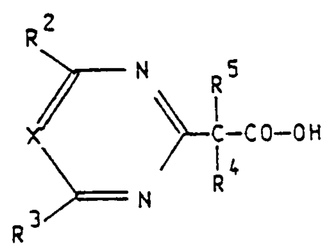

(VI)

dans laquelle $R^2$, $R^3$, $R^4$, $R^5$ et X ont les significations données dans la formule générale I, avec une base appropriée, dans un solvant approprié pour obtenir des composés de formule générale VII

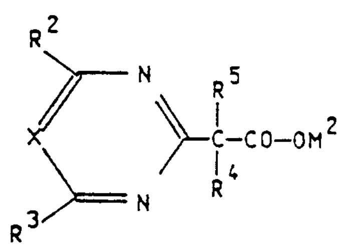

(VII)

dans laquelle $R^2$, $R^3$, $R^4$, $R^5$ et X ont les significations données pour la formule générale I et $M^2$ représente un atome de métal alcalin, un équivalent d'un atome de métal alcalino-terreux, un ion ammonium ou un groupe ammonium substitué par des radicaux organiques, ou

E) on fait réagir un composé de formule générale VIII

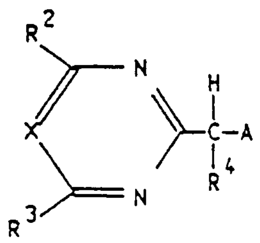

(VIII)

dans laquelle A, $R^2$, $R^3$, $R^4$ et X ont les significations données pour la formule générale I, avec un composé de formule générale IX

$$R^5 - Z \qquad (IX)$$

dans laquelle $R^5$ a les significations données pour la formule générale I et Z représente un atome d'halogène, un groupe alkylsulfonyloxy, un groupe phénylsulfonyloxy ou un groupe $-O-SO_2-O$-alkyle, dans un solvant approprié, en présence d'une base appropriée.

3. Agent ayant un effet herbicide, fongicide ou régulateur de croissance végétale, caractérisé par une teneur en au moins un composé selon la revendication 1.

4. Utilisation des agents selon la revendication 3, pour la lutte contre les plantes adventices mono- et dicotylées dans les cultures agricoles.

5. Utilisation des agents selon la revendication 3, pour la lutte contre une attaque par des champignons des cultures agricoles.

6. Utilisation des agents selon la revendication 3, pour influencer la croissance des plantes de culture.

7. Procédé pour la préparation d'agents ayant un effet herbicide, fongicide et régulateur de croissance, caractérisé en ce qu'on mélange des composés de formule générale I, selon la revendication 1 avec des véhicules ou adjuvants.

8. 2-(4,6-diméthoxy-pyrimidin-2-yl)-3-cyclopropyl)-propanoate de méthyle.